(19) Europäisches Patentamt
European Patent Office
Office européen des brevets

(11) **EP 2 962 283 B1**

(12) **EUROPEAN PATENT SPECIFICATION**

(45) Date of publication and mention of the grant of the patent:
**25.09.2019 Bulletin 2019/39**

(21) Application number: **14712837.5**

(22) Date of filing: **28.02.2014**

(51) Int Cl.:
*G06T 7/20* (2017.01)      *G06T 7/00* (2017.01)
*A61B 5/00* (2006.01)      *A61B 6/00* (2006.01)
*A61B 8/08* (2006.01)      *A61B 5/02* (2006.01)

(86) International application number:
**PCT/US2014/019428**

(87) International publication number:
**WO 2014/134454 (04.09.2014 Gazette 2014/36)**

(54) **SYSTEMS AND METHODS FOR LUMEN BORDER DETECTION IN INTRAVASCULAR ULTRASOUND SEQUENCES**

SYSTEME UND VERFAHREN ZUR LUMENGRENZENERKENNUNG IN INTRAVASKULAREN ULTRASCHALLSEQUENZEN

SYSTÈMES ET PROCÉDÉS POUR LA DÉTECTION DE BORDURE DE LUMIÈRE DANS DES SÉQUENCES D'ULTRASONS INTRAVASCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2013 US 201361771285 P**

(43) Date of publication of application:
**06.01.2016 Bulletin 2016/01**

(73) Proprietor: **Boston Scientific Scimed, Inc.**
**Maple Grove, MN 55311 (US)**

(72) Inventors:
• **CAI, Anming, He**
  **San Jose, CA 95129 (US)**
• **LI, Wenguang**
  **Santa Clara, CA 95054 (US)**
• **SATHYANARAYANA, Shashidhar**
  **Pleasanton, CA 94566 (US)**
• **THOMAS, Lewis, Jones, III**
  **Palo Alto, CA 94306 (US)**

(74) Representative: **Vossius & Partner**
**Patentanwälte Rechtsanwälte mbB**
**Siebertstrasse 3**
**81675 München (DE)**

(56) References cited:
**US-A- 6 152 878**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention is directed to the area of imaging systems that are insertable into a patient and methods of analyzing ultrasound images obtained therefrom. The present invention is also directed to methods and imaging systems for lumen border detection in intravascular ultrasound sequences.

BACKGROUND

Ultrasound devices insertable into patients have proven diagnostic capabilities for a variety of diseases and disorders. For example, intravascular ultrasound ("IVUS") imaging systems have been used as an imaging modality for diagnosing blocked blood vessels and providing information to aid medical practitioners in selecting and placing stents and other devices to restore or increase blood flow. IVUS imaging systems have been used to diagnose atheromatous plaque build-up at particular locations within blood vessels. IVUS imaging systems can be used to determine the existence of an intravascular obstruction or stenosis, as well as the nature and degree of the obstruction or stenosis. IVUS imaging systems can be used to visualize segments of a vascular system that may be difficult to visualize using other intravascular imaging techniques, such as angiography, due to, for example, movement (*e.g.*, a beating heart) or obstruction by one or more structures (*e.g.*, one or more blood vessels not desired to be imaged). IVUS imaging systems can be used to monitor or assess ongoing intravascular treatments, such as angiography and stent placement in real (or almost real) time. Moreover, IVUS imaging systems can be used to monitor one or more heart chambers.

IVUS imaging systems have been developed to provide a diagnostic tool for visualizing a variety of diseases or disorders. An IVUS imaging system can include a control module (with a pulse generator, an image processor, and a monitor), a catheter, and one or more transducers disposed in the catheter. The transducer-containing catheter can be positioned in a lumen or cavity within, or in proximity to, a region to be imaged, such as a blood vessel wall or patient tissue in proximity to a blood vessel wall. The pulse generator in the control module generates electrical pulses that are delivered to the one or more transducers and transformed to acoustic pulses that are transmitted through patient tissue. Reflected pulses of the transmitted acoustic pulses are absorbed by the one or more transducers and transformed to electric pulses. The transformed electric pulses are delivered to the image processor and converted to an image displayable on the monitor. There is a need for systems and methods for identifying the vascular lumen in a sequence of IVUS images to allow a practitioner to evaluate the sequence of images.

US 6,152,878 A discloses a device and method for intravascular ultrasound imaging. A catheter including ultrasonic apparatus is introduced into a bodily lumen. The apparatus transmits ultrasonic signals and detects reflected ultrasound signals which contain information relating to the bodily lumen. A processor coupled to the catheter is programmed to derive a first image or series of images and a second image or series of images from the detected ultrasound signals. The processor is also programmed to compare the second image or series of images to the first image or series of images respectively.

BRIEF SUMMARY

One embodiment is a method for processing a sequence of ultrasound frames for display. The method includes receiving a sequence of intravascular ultrasound (IVUS) frames of a vessel having a lumen, the sequence including a first frame and a second frame; determining one or more texture features for each of one or more regions of the first frame; determining at least one flow feature for each of the one or more regions by comparing the first and second frames; deriving a lumen border for the first frame using the one or more texture features and the at least one flow feature to characterize the one or more regions as within or outside of the lumen of the vessel; and displaying an ultrasound image of the first frame with the lumen border.

Another embodiment is a non-transitory computer-readable medium having processor-executable instructions for processing a sequence of ultrasound frames. The processor-executable instructions when installed onto a device enable the device to perform actions including receiving a sequence of intravascular ultrasound (IVUS) frames of a vessel having a lumen, the sequence including a first frame and a second frame; determining one or more texture features for each of one or more regions of the first frame; determining at least one flow feature for each of the one or more regions by comparing the first and second frames; deriving a lumen border for the first frame using the one or more texture features and the at least one flow feature to characterize the one or more regions as within or outside of the lumen of the vessel; and displaying an ultrasound image of the first frame with the lumen border.

Yet another embodiment is a system for generating and processing a sequence of ultrasound frames. The system includes a catheter and an ultrasound imaging core insertable into the catheter. The ultrasound imaging core includes at least one transducer and is configured and arranged for rotation of at least a portion of the ultrasound imaging core to provide a sequence of ultrasound frames. The system also includes a processor, coupleable to the ultrasound imaging core, for executing processor-readable instructions that enable actions including receiving a sequence of intravascular ultrasound (IVUS) frames of a vessel having a lumen, the sequence including a first frame and a second frame; determining one or more texture features for each of one or more regions of the first frame; determining at least one flow feature for each of the one or more regions by comparing the first and second frames; deriving a lumen border for the first frame using the one or more texture features and the at least one flow feature to characterize the one or more regions as within or outside of the lumen of the vessel; and displaying an ultrasound image of the first frame with the lumen border.

BRIEF DESCRIPTION OF THE DRAWINGS

Non-limiting and non-exhaustive embodiments of the present invention are described with reference to the following drawings. In the drawings, like reference numerals refer to like parts throughout the various figures unless otherwise specified.

For a better understanding of the present invention, reference will be made to the following Detailed Description, which is to be read in association with the accompanying drawings, wherein:

FIG. 1 is a schematic view of one embodiment of an ultrasound imaging system suitable for insertion into a patient, according to the invention;

FIG. 2 is a schematic side view of one embodiment of a catheter suitable for use with the ultrasound imaging system of FIG. 1, according to the invention;

FIG. 3 is a schematic longitudinal cross-sectional view of one embodiment of a distal end of the catheter of FIG. 2 with an imaging core disposed in a lumen defined in a sheath, according to the invention;

FIG. 4 is a schematic flow chart of one embodiment of a method of processing a sequence of ultrasound images for lumen border detection, according to the invention; and

FIG. 5 is a schematic flow diagram of another embodiment of a method of processing a sequence of ultrasound images for lumen border detection, according to the invention.

DETAILED DESCRIPTION

The present invention is directed to the area of imaging systems that are insertable into a patient and methods of analyzing ultrasound images obtained therefrom. The present invention is also directed to methods and imaging systems for lumen border detection in intravascular ultrasound sequences.

The methods, systems, and devices described herein may be embodied in many different forms and should not be construed as limited to the embodiments set forth herein. Accordingly, the methods, systems, and devices described herein may take the form of an entirely hardware embodiment, an entirely software embodiment, or an embodiment combining software and hardware aspects. The methods described herein can be performed using any type of computing device, such as a computer, that includes a processor or any combination of computing devices where each device performs at least part of the process.

Suitable computing devices typically include mass memory and typically include communication between devices. The mass memory illustrates a type of computer-readable media, namely computer-readable storage media. Computer-readable storage media may include volatile, nonvolatile, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computing device.

Methods of communication between devices or components of a system or between the system and other devices can include both wired and wireless (e.g., RF, optical, or infrared) communications methods and such methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, and other wireless media.

Suitable intravascular ultrasound ("IVUS") imaging systems include, but are not limited to, one or more transducers disposed on a distal end of a catheter configured and arranged for percutaneous insertion into a patient. Examples of IVUS imaging systems with catheters are found in, for example, U.S. Patents Nos. 6,945,938; 7,246,959; and 7,306,561; as well as U.S. Patent Application Publication Nos. 2006/0100522; 2006/0106320; 2006/0173350; 2006/0253028; 2007/0016054; and 2007/0038111.

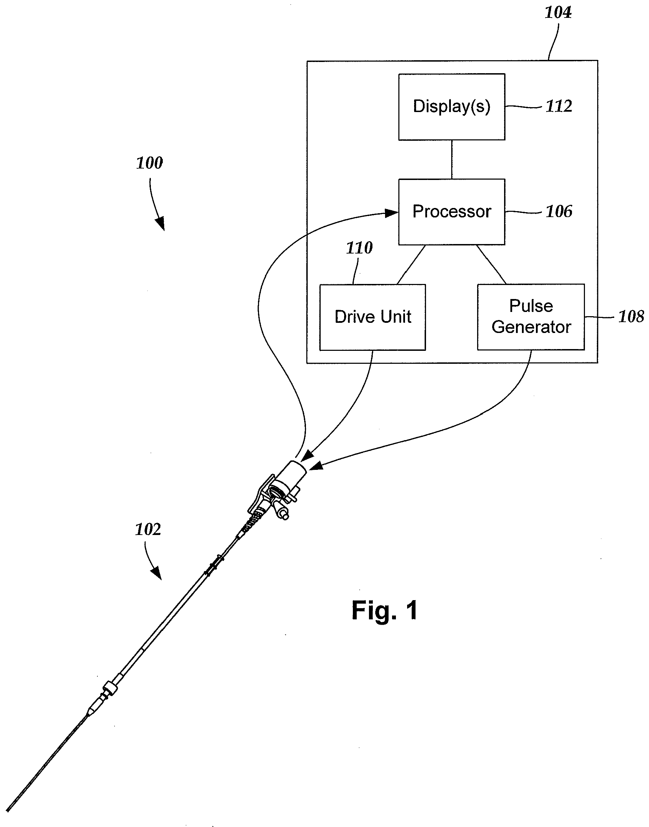

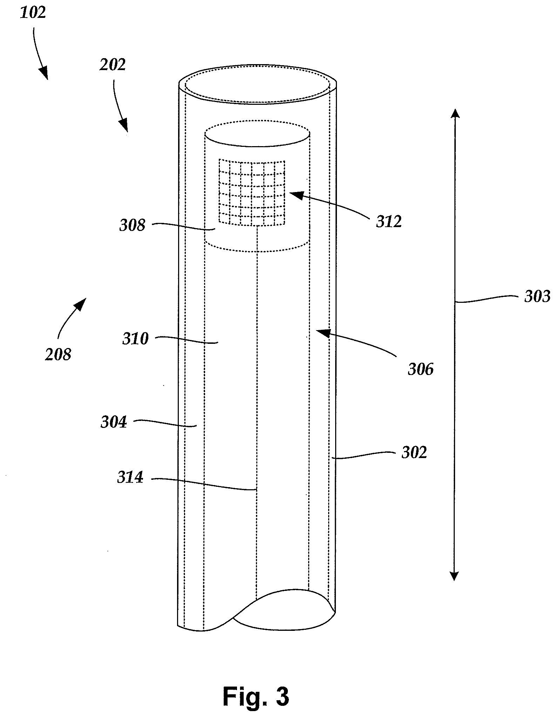

Figure 1 illustrates schematically one embodiment of an IVUS imaging system 100. The IVUS imaging system 100 includes a catheter 102 that is coupleable to a control module 104. The control module 104 may include, for example, a processor 106, a pulse generator 108, a drive unit 110, and one or more displays 112. In at least some embodiments, the pulse generator 108 forms electric pulses that may be input to one or more transducers (312 in Figure 3) disposed in the catheter 102.

In at least some embodiments, mechanical energy from the drive unit 110 may be used to drive an imaging core (306 in Figure 3) disposed in the catheter 102. In at least some embodiments, electric signals transmitted from the one or more transducers (312 in Figure 3) may be input to the processor 106 for processing. In at least some embodiments, the processed electric signals from the one or more transducers (312 in Figure 3) can be displayed as one or more images on the one or more displays 112. For example, a scan converter can be used to map scan line samples (e.g., radial scan line samples, or the like) to a two-dimensional Cartesian grid to display the one or more images on the one or more displays 112.

In at least some embodiments, the processor 106 may also be used to control the functioning of one or more of the other components of the control module 104. For example, the processor 106 may be used to control at least one of the frequency or duration of the electrical pulses transmitted from the pulse generator 108, the rotation rate of the imaging core (306 in Figure 3) by the drive unit 110, the velocity or length of the pullback of the imaging core (306 in Figure 3) by the drive unit 110, or one or more properties of one or more images formed on the one or more displays 112.

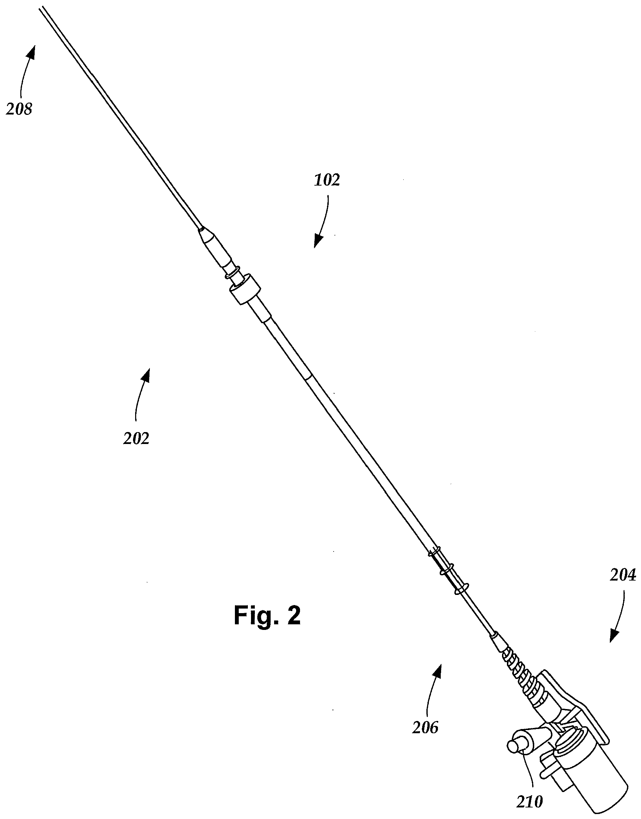

Figure 2 is a schematic side view of one embodiment of the catheter 102 of the IVUS imaging system (100 in Figure 1). The catheter 102 includes an elongated member 202 and a hub 204. The elongated member 202 includes a proximal end 206 and a distal end 208. In Figure 2, the proximal end 206 of the elongated member 202 is coupled to the catheter hub 204 and the distal end 208 of the elongated member is configured and arranged for percutaneous insertion into a patient. Optionally, the catheter 102 may define at least one flush port, such as flush port 210. The flush port 210 may be defined in the hub 204. The hub 204 may be configured and arranged to couple to the control module (104 in Figure 1). In some embodiments, the elongated member 202 and the hub 204 are formed as a unitary body. In other embodiments, the elongated member 202 and the catheter hub 204 are formed separately and subsequently assembled together.

Figure 3 is a schematic perspective view of one embodiment of the distal end 208 of the elongated member 202 of the catheter 102. The elongated member 202 includes a sheath 302 with a longitudinal axis 303 and a lumen 304. An imaging core 306 is disposed in the lumen 304. The imaging core 306 includes an imaging device 308 coupled to a distal end of a driveshaft 310 that is rotatable either manually or using a computer-controlled drive mechanism. One or more transducers 312 may be mounted to the imaging device 308 and employed to transmit and receive acoustic signals. The sheath 302 may be formed from any flexible, biocompatible material suitable for insertion into a patient. Examples of suitable materials include, for example, polyethylene, polyurethane, plastic, spiral-cut stainless steel, nitinol hypotube, and the like or combinations thereof.

In a preferred embodiment (as shown in Figure 3), an array of transducers 312 are mounted to the imaging device 308. In alternate embodiments, a single transducer may be employed. Any suitable number of transducers 312 can be used. For example, there can be two, three, four, five, six, seven, eight, nine, ten, twelve, fifteen, sixteen, twenty, twenty-five, fifty, one hundred, five hundred, one thousand, or more transducers. As will be recognized, other numbers of transducers may also be used. When a plurality of transducers 312 are employed, the transducers 312 can be configured into any suitable arrangement including, for example, an annular arrangement, a rectangular arrangement, or the like.

The one or more transducers 312 may be formed from one or more known materials capable of transforming applied electrical pulses to pressure distortions on the surface of the one or more transducers 312, and vice versa. Examples of suitable materials include piezoelectric ceramic materials, piezocomposite materials, piezoelectric plastics, barium titanates, lead zirconate titanates, lead metaniobates, polyvinylidenefluorides, and the like. Other transducer technologies include composite materials, single-crystal composites, and semiconductor devices (e.g., capacitive micromachined ultrasound transducers ("cMUT"), piezoelectric micromachined ultrasound transducers ("pMUT"), or the like)

The pressure distortions on the surface of the one or more transducers 312 form acoustic pulses of a frequency based on the resonant frequencies of the one or more transducers 312. The resonant frequencies of the one or more transducers 312 may be affected by the size, shape, and material used to form the one or more transducers 312. The one or more transducers 312 may be formed in any shape suitable for positioning within the catheter 102 and for propagating acoustic pulses of a desired frequency in one or more selected directions. For example, transducers may be disc-shaped, block-shaped, rectangular-shaped, oval-shaped, and the like. The one or more transducers may be formed in the desired shape by any process including, for example, dicing, dice and fill, machining, microfabrication, and the like.

As an example, each of the one or more transducers 312 may include a layer of piezoelectric material sandwiched between a matching layer and a conductive backing material formed from an acoustically absorbent material (*e.g.*, an epoxy substrate with tungsten particles). During operation, the piezoelectric layer may be electrically excited to cause the emission of acoustic pulses.

The one or more transducers 312 can be used to form a radial cross-sectional image of a surrounding space. Thus, for example, when the one or more transducers 312 are disposed in the catheter 102 and inserted into a blood vessel of a patient, the one more transducers 312 may be used to form an image of the walls of the blood vessel and tissue surrounding the blood vessel.

The imaging core 306 is rotated about the longitudinal axis 303 of the catheter 102. As the imaging core 306 rotates, the one or more transducers 312 emit acoustic signals in different radial directions (*i.e.,* along different radial scan lines). For example, the one or more transducers 312 can emit acoustic signals at regular (or irregular) increments, such as 256 radial scan lines per revolution, or the like. It will be understood that other numbers of radial scan lines can be emitted per revolution, instead.

When an emitted acoustic pulse with sufficient energy encounters one or more medium boundaries, such as one or more tissue boundaries, a portion of the emitted acoustic pulse is reflected back to the emitting transducer as an echo pulse. Each echo pulse that reaches a transducer with sufficient energy to be detected is transformed to an electrical signal in the receiving transducer. The one or more transformed electrical signals are transmitted to the control module (104 in Figure 1) where the processor 106 processes the electrical-signal characteristics to form a displayable image of the imaged region based, at least in part, on a collection of information from each of the acoustic pulses transmitted and the echo pulses received. In at least some embodiments, the rotation of the imaging core 306 is driven by the drive unit 110 disposed in the control module (104 in Figure 1). In alternate embodiments, the one or more transducers 312 are fixed in place and do not rotate. In which case, the driveshaft 310 may, instead, rotate a mirror that reflects acoustic signals to and from the fixed one or more transducers 312.

When the one or more transducers 312 are rotated about the longitudinal axis 303 of the catheter 102 emitting acoustic pulses, a plurality of images can be formed that collectively form a radial cross-sectional image (*e.g.,* a tomographic image) of a portion of the region surrounding the one or more transducers 312, such as the walls of a blood vessel of interest and tissue surrounding the blood vessel. The radial cross-sectional image can, optionally, be displayed on one or more displays 112. The at least one of the imaging core 306 can be either manually rotated or rotated using a computer-controlled mechanism.

The imaging core 306 may also move longitudinally along the blood vessel within which the catheter 102 is inserted so that a plurality of cross-sectional images may be formed along a longitudinal length of the blood vessel. During an imaging procedure the one or more transducers 312 may be retracted (*i.e.,* pulled back) along the longitudinal length of the catheter 102. The catheter 102 can include at least one telescoping section that can be retracted during pullback of the one or more transducers 312. In at least some embodiments, the drive unit 110 drives the pullback of the imaging core 306 within the catheter 102. The drive unit 110 pullback distance of the imaging core can be any suitable distance including, for example, at least 5 cm, 10 cm, 15 cm, 20 cm, 25 cm, or more. The entire catheter 102 can be retracted during an imaging procedure either with or without the imaging core 306 moving longitudinally independently of the catheter 102.

A stepper motor may, optionally, be used to pull back the imaging core 306. The stepper motor can pull back the imaging core 306 a short distance and stop long enough for the one or more transducers 306 to capture an image or series of images before pulling back the imaging core 306 another short distance and again capturing another image or series of images, and so on.

The quality of an image produced at different depths from the one or more transducers 312 may be affected by one or more factors including, for example, bandwidth, transducer focus, beam pattern, as well as the frequency of the acoustic pulse. The frequency of the acoustic pulse output from the one or more transducers 312 may also affect the penetration depth of the acoustic pulse output from the one or more transducers 312. In general, as the frequency of an acoustic pulse is lowered, the depth of the penetration of the acoustic pulse within patient tissue increases. In at least some embodiments, the IVUS imaging system 100 operates within a frequency range of 5 MHz to 100 MHz.

One or more conductors 314 can electrically couple the transducers 312 to the control module 104 (see *e.g.,* Figure 1). In which case, the one or more conductors 314 may extend along a longitudinal length of the rotatable driveshaft 310.

The catheter 102 with one or more transducers 312 mounted to the distal end 208 of the imaging core 308 may be inserted percutaneously into a patient via an accessible blood vessel, such as the femoral artery, femoral vein, or jugular vein, at a site remote from the selected portion of the selected region, such as a blood vessel, to be imaged. The catheter 102 may then be advanced through the blood vessels of the patient to the selected imaging site, such as a portion of a selected blood vessel.

An image frame ("frame") of a composite image can be generated each time one or more acoustic signals are output to surrounding tissue and one or more corresponding echo signals are received by the imager 308 and transmitted to the processor 106. A plurality (e.g., a sequence) of frames may be acquired over time during any type of movement of the imaging device 308. For example, the frames can be acquired during rotation and pullback of the imaging device 308 along the target imaging location. It will be understood that frames may be acquired both with or without rotation and with or without pullback of the imaging device 308. Moreover, it will be understood that frames may be acquired using other types of movement procedures in addition to, or in lieu of, at least one of rotation or pullback of the imaging device 308.

In at least some embodiments, when pullback is performed, the pullback may be at a constant rate, thus providing a tool for potential applications able to compute longitudinal vessel/plaque measurements. In at least some embodiments, the imaging device 308 is pulled back at a constant rate of at least 0.3 mm/s. In at least some embodiments, the imaging device 308 is pulled back at a constant rate of at least 0.4 mm/s. In at least some embodiments, the imaging device 308 is pulled back at a constant rate of at least 0.5 mm/s. In at least some embodiments, the imaging device 308 is pulled back at a constant rate of at least 0.6 mm/s. In at least some embodiments, the imaging device 308 is pulled back at a constant rate of at least 0.7 mm/s. In at least some embodiments, the imaging device 308 is pulled back at a constant rate of at least 0.8 mm/s.

In at least some embodiments, the one or more acoustic signals are output to surrounding tissue at constant intervals of time. In at least some embodiments, the one or more corresponding echo signals are received by the imager 308 and transmitted to the processor 106 at constant intervals of time. In at least some embodiments, the resulting frames are generated at constant intervals of time.

A system can automatically extract lumen geometric information from intravascular ultrasound images. The system can exploit the differences between lumen and vessel wall in texture, ultrasound frequency response, and changes between consecutive image frames, to determine each image pixel's (or some subset of image pixel's) probability of being inside the lumen border. The border can be drawn to illustrate the lumen accounting for lumen probability inside the border with predefined curvature constraints. The systems and methods of lumen border detection described herein can be used with the IVUS systems and catheters described above or with any other IVUS imaging system, catheter, or device.

Intravascular ultrasound (IVUS) imaging provides a cross-sectional view of blood vessels to aid in visualization of, for example, vascular diseases such as coronary arterial narrowing. The vascular lumen border is one clinically relevant quantitative measurement in IVUS. Currently, existing devices that provide automatic lumen border detection require user to make substantial manual corrections to accurately determine the lumen border. As manual measurements may take significant time during the IVUS procedure, a fully automated system with high accuracy will shorten the procedure time, improve the workflow and provide a mare accurate estimate than the "eyeballing" method that is often used in order to save time. Although attempts have been made to develop fully automatic lumen detection, no method has been proved to provide sufficient accuracy in a large patient database.

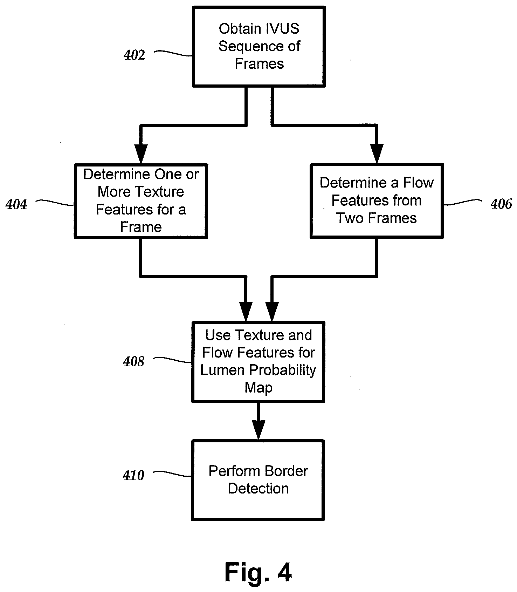

Figure 4 is a flowchart illustrating one example of an automatic method and system for border detection. First, an IVUS sequence of frames is obtained (step 402). The sequence of frames can be obtained from an IVUS imaging device (e.g., catheter) that is part of the system or the sequence of frames can be obtained from another source and transferred to the system using any transfer method including, but not limited to, wireless or wired transfer or transfer using a computer readable medium such as a compact disc, flash memory, or any other suitable medium.

For each frame, or for a subset of frames, one or more texture features are determined for pixels, or for regions of pixels, within the frame (step 404). The one or more texture features can be, for example, filtered or averaged values associated with a pixel, or region, and one or more of its neighbors. The texture features exploit the differences in ultrasound response (e.g., differences in intensity or frequency) between tissue types and between tissue and blood (which flows through the lumen.)

A region of the image, rather than a single pixel, can be selected for determination of the texture feature. Furthermore, the texture feature(s) for all of the pixels, or regions, in a frame can be determined or the texture feature(s) can be determined for a subset of the pixels, or regions, of each frame. This subset of pixels, or regions, may be manually selected by a user or may be automatically selected by the system (for example, by selecting pixels or regions which exceed a certain intensity threshold or fall within a certain intensity range or are within a particular portion of the image or any combination thereof).

The texture features can be determined using one or more filters to reduce the effect of noise and imaging anomalies. The texture feature examples provided below utilize the pixel intensity in determining the feature value, but it will be recognized that other image or pixel properties can be used instead of, or in combination with, pixel intensity.

One type of texture feature is determined using a cross-line, or lateral, filter that combines the values of pixels at the same position in neighboring scan lines. The scan lines can be adjacent to each other or can be separated by one or more scan lines. Examples of suitable lateral filters have one of the following formulas: $F_{i,n} = |I_{i,n}-I_{i,n+3}|$ or $F_{i,n} = |I_{i,n}-I_{i,n+4}|$ where $I_n$ is the intensity of pixel i in scan line n and $F_{i,n}$ is the texture value for pixel i in scan line n. These particular filters are useful for border detection because pixels in the two scan lines that are within the same tissue will result in a texture value near 0 while those pixels straddling a border between different tissue types or between tissue and lumen will have values substantially greater than 0. The texture values may also be smoothed over a surrounding region using, for example, a boxcar filter. The texture values may also be normalized. The texture values may be used as the feature value or may be used in calculating the feature value.

Another type of texture feature is determined using a cross-depth, or axial, filter that combines the values of neighboring pixels along the same scan line. One example of this axial filter has the following formula: $F_{1,n} = |I_{i,n}-I_{i+3,n}|$ where $I_n$ is the intensity of pixel i in scan line n and $F_{i,n}$ is the texture value for pixel i in scan line n. These particular filters are useful for border detection because pixels at the two positions along the scan line that are within the same tissue will result in a value near 0 while those pixels straddling a border between different tissue types or between tissue and lumen will have values substantially greater than 0. The texture values may also be smoothed over a surrounding region using, for example, a boxcar filter. The texture values may also be normalized. The texture values may be used as the feature value or may be used in calculating the feature value.

In at least one embodiment, two texture features are determined. One of the texture features is determined using a lateral filter and the other texture feature is determined using an axial filter.

In addition, for each frame, or for a subset of frames, at least one flow feature is determined for pixels, or for regions of pixels, within the frames (step 406). The flow feature(s) for all of the pixels, or regions, in a frame can be determined or the texture feature(s) can be determined for a subset of the pixels, or regions, of each frame. This subset of pixels, or regions, may be manually selected by a user or may be automatically selected by the system (for example, by selecting pixels or regions which exceed a certain intensity threshold or fall within a certain intensity range or are within a particular portion of the image or any combination thereof).

The flow feature(s) is determined by comparing corresponding portions of two or more frames. The two frames being compared can be two adjacent frames within the sequence or the two frames that are separated by one or more frames within the sequence.

In at least some embodiments, a flow feature is determined using cross-frame correlation. In general, tissue regions will show relatively high correlation between frames, but blood regions (i.e., the lumen) will have substantially lower correlation between frames due, at least in part, to blood flow. One complication with determining correlation between two frames is that there may be movement of the tissue between frames. Accordingly, in at least some embodiments, the correlation calculation may compare a region in the first frame with a larger region in the second frame in order to adjust for the movement.

As an example, for a pixel (x,y) of the first frame a correlation window $W_c$ can be defined as centered around the pixel (x,y) with widths $w_x$, $w_y$ in the corresponding axes. (It will be recognized that other correlation windows can be selected that are not necessarily centered around the pixel.) For example, the correlation window could be a 7x7 window centered around the pixel (x,y).

A corresponding search window $W_s$ can be defined in the second frame. This search window can be centered around the corresponding pixel (x,y) in the second frame or around another pixel, if desired (for example, if the movement of the initial region is known or approximated.) In at least some embodiments, the widths of the search window $W_s$ are $w_x+2s_x$, $w_y+2s_y$ where $s_x$, $s_y$ are the expansion of the boundary of the search window relative to the correlation window. For example, the correlation window $W_c$ could be a 7x7 window centered around the pixel (x,y) in the first frame and the search window $W_s$ could be a 11x11 window centered around the pixel (x,y) in the second frame. (Again, it will be recognized that other correlation windows can be selected that are not necessarily centered around the pixel.)

One or more correlation values between the correlation window of the first frame and the search window of the second frame can be determined using any method of calculation. One example of a determination of a set of correlation values $C_n(x, y; \delta_x, \delta_y)$, where $\delta_x \in [-s_x, s_x]$ and $\delta_y \in [-s_y, s_y]$, for pixel (x,y) of first frame n and second frame n+1 uses the following set of equations:

$$C_n(x,y;\delta_x,\delta_y) = \frac{\sum_{(i,j)\in W_c}[I_n(x+i,y+j)-\bar{I}_n(x,y)]\cdot[I_{n+1}(x+\delta_x+i,y+\delta_y+j)-\bar{I}_{n+1}(x+\delta_x,y+\delta_y)]}{A^2\sqrt{\bar{I}_n^2(x,y)-(\bar{I}_n(x,y))^2}\cdot\sqrt{\bar{I}_{n+1}^2(x+\delta_x,y+\delta_y)-(\bar{I}_{n+1}(x+\delta_x,y+\delta_y))^2}}$$

$$A = \sum_{(i,j)\in W_c} 1 = w_x \cdot w_y, \quad \bar{I}_n(x,y) = \frac{1}{A}\sum_{(i,j)\in W_c} I_n(x+i, y+j), \quad \bar{I}_n^2(x,y) = \frac{1}{A}\sum_{(i,j)\in W_c}(I_n(x+i, y+j))^2.$$

where the flow feature can then be determined using the maximum of the set of correlation values:

$$C_n^{\max}(x,y) = \max(C_n(x,y; \delta_x \in [-s_x, s_x], \delta_y \in [-s_y, s_y])).$$

The texture and flow features can then be used to determine a lumen probability map (step 408) or other analytical arrangement that represents the probability that the corresponding pixel, or region, corresponds to the lumen of the vessel. For example, the values of the texture and flow features can be compared to typical values of those features for lumen and non-lumen regions to aid in determining the lumen probability map.

Such comparisons may be qualitative or quantitative. Quantitative comparisons may include, for example, determining a distance, in feature space, from the average of feature values for the vessel or feature values for the lumen (or determining both a distance relative to the lumen values and a distance relative to the vessel values). This distance calculation may include weighting some features more than others. Such weighting may include empirically derived weights. The weighting may also depend on factors such as, for example, the apparatus used to obtain the sequence of frames, the type of vessel being observed, and the like.

In at least some embodiments, a value can be assigned to the probability that the pixel or region is within the lumen. For example, the values may range from -1 (e.g., definitely outside the lumen) to +1 (e.g., definitely within the lumen) or over any other suitable range (e.g., 0 to 100 or 0 to 1).

Once the lumen probability map is determined, the border can be detected or determined (step 410). For example, the border can be drawn that maximizes total lumen probability within the border. Drawing constraints, such as a penalty for deviation from an expected border geometry (e.g., a quadratic interpolation), can be used to smooth the border. As another example, the border may be drawn around a region that has a lumen probability above (or below) a particular threshold value. Any other suitable method for determining the border of the lumen from the lumen probability map may be used. In at least some instances, the lumen probability map may be smoothed prior to border detection.

Determination of the lumen border can facilitate determination of other clinically relevant measurements including, for example, lumen cross-section area (the area bounded by the luminal border); minimum lumen diameter, $D_{min}$; maximum lumen diameter, $D_{max}$; and lumen eccentricity, $(D_{max}-D_{min})/D_{max}$. In addition, determination of the lumen border through multiple frames obtained during pullback of the imaging core can facilitate comparison of these measurements along the vessel and can also be used to create a three dimensional view of the vessel. From the multi-frame determination of the lumen border, the minimal lumen area along the imaged portion of the vessel can be determined.

Other vessel information can be determined including for example, a proximal reference site with the largest lumen proximal to a stenosis but still within the same vessel segment (usually within 10 mm of the stenosis with no intervening branches), a distal reference site with the largest lumen proximal to a stenosis but still within the same vessel segment (usually within 10 mm of the stenosis with no intervening branches), the largest reference of either the proximal or distal reference sites, and the average reference lumen size (i.e., the average of the lumen size at the proximal reference site and the distal reference site.) From this, the lumen area stenosis can be calculated as, for example, (reference cross-section area - minimum lumen cross-section area)/reference cross-section area. This lumen area stenosis can be classified as proximal, distal, largest, or averaged depending on which reference cross-section area is chosen for the calculation.

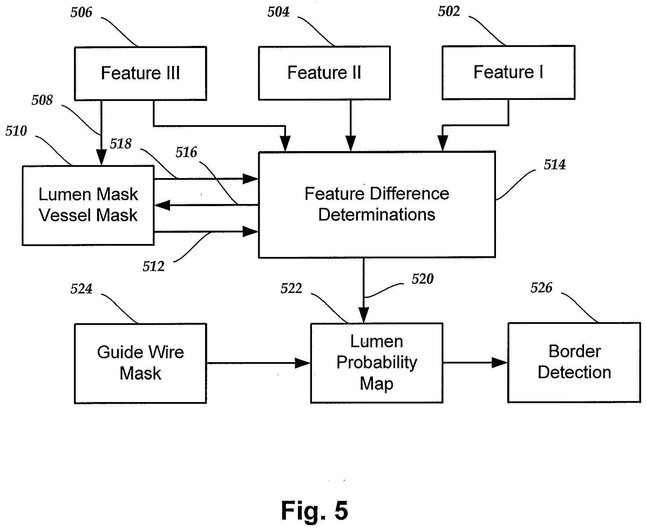

The process described above can be further refined using iterative and other techniques. Figure 5 illustrates one example of another process for border detection. In this example, Feature I 502 and Feature II 504 are texture features obtained using, for example, a lateral filter and an axial filter, respectively, as described above. Feature III 506 is a flow feature obtained using, for example, cross-frame correlation. It will be recognized that other types of texture and flow features can be used for Feature I, Feature II, and Feature III.

Feature III 506 is used to estimate (arrow 508) lumen or vessel masks 510 (or both lumen and vessel masks). For example, a lumen mask may be estimated to be those regions that have a correlation value below a threshold value (e.g., 0.2 or 0.3 for a normalized calculation) and the vessel mask may be those regions where the correlation value is above the threshold value.

The lumen/vessel masks may be used (arrow 512) with Feature I 502, Feature II 504, and Feature III 506 to make feature difference determinations 514 in feature space (e.g., feature distance determination such as the distance from a pixel or region to the lumen mask or to the vessel mask in a three-dimensional feature space with the three features corresponding to the three dimensions). These feature difference determinations 514 can then be used (arrow 516) to refine the lumen/vessel masks 510. The refined lumen/vessel masks can then be used (arrow 518), with Features I, II, and III, to refine the feature difference determinations. It will be understood that further iterative refinement of the lumen/vessel masks and feature difference determinations can be implemented.

The refined feature difference determinations (for example, feature distance determinations in feature space) can then be used (arrow 520) to determine the lumen probability map 522 as described above. Optionally, a guide wire mask 524 may also be introduced to aid in determining the lumen probability map. As further described above, border detection 526 can be performed using the lumen probability map.

It will be understood that each block of the block diagram illustrations, and combinations of blocks in the block diagram illustrations, as well any portion of the systems and methods disclosed herein, can be implemented by computer program instructions. These program instructions may be provided to a processor to produce a machine, such that the instructions, which execute on the processor, create means for implementing the actions specified in the block diagram block or blocks or described for the systems and methods disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system. In addition, one or more processes may also be performed concurrently with other processes, or even in a different sequence than illustrated without departing from the scope of the invention.

The computer program instructions can be stored on any suitable computer-readable medium including, but not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computing device.

The above specification, examples and data provide a description of the manufacture and use of the composition of the invention. Since many embodiments of the invention can be made without departing from the scope of the invention, the invention also resides in the claims hereinafter appended.

### Claims

1.  A method for processing a sequence of ultrasound frames for display, the method comprising:

    receiving a sequence of intravascular ultrasound, IVUS, frames of a vessel having a lumen, the sequence comprising a first frame and a second frame, wherein each frame comprises a plurality of scan lines, each scan line comprising a plurality of pixels;
    determining one or more texture features for each of one or more regions of the first frame, wherein at least one of the one or more texture features comprises a difference between intensity values of i) the region of the first frame and ii) another region of the first frame spaced apart from the region;
    determining at least one flow feature using cross-frame correlation for each of the one or more regions by comparing the first and second frames;
    deriving a lumen border for the first frame using the one or more texture features and the at least one flow feature to characterize the one or more regions as within or outside of the lumen of the vessel; and
    displaying an ultrasound image of the first frame with the lumen border;
    wherein the one or more regions are each a pixel of the first frame.

2.  The method of claim 1, wherein determining one or more texture features comprises determining the difference in intensity values from pixels of two or more different scan lines of the first frame, wherein determining the difference in intensity values optionally comprises determining the difference in intensity values from pixels of two or more different non-adjacent scan lines.

3.  The method of claim 1, wherein determining one or more texture features comprises determining the difference in intensity values from at least two pixels of a single scan line of the first frame, wherein determining the difference in intensity values optionally comprises determining the difference in intensity values from at least two non-adjacent pixels of a single scan line.

4.  The method of claim 1, wherein the sequence further comprises at least one additional frame and the method further comprises, for each of the at least one additional frame,
    determining one or more texture features for each of one or more regions of the additional frame, wherein at least one of the one or more texture features comprises a difference between intensity values of i) the region of the additional IVUS frame and ii) another region of the additional IVUS frame spaced apart from the region;
    determining at least one flow feature for each of the one or more regions by comparing the additional frame with another frame in the sequence; and
    deriving a lumen border for the additional frame using the one or more texture features and the at least one flow feature to characterize the one or more regions as within or outside of the lumen of the vessel; and

displaying an ultrasound image of the additional frame with the lumen border derived for the additional frame; wherein the one or more regions are each a pixel.

5. The method of claim 1, wherein determining at least one flow feature comprises determining a cross-frame flow feature by determining a correlation value between a correlation window in the first frame and a search window in the second frame.

6. The method of claim 5, wherein the search window has a larger area then the correlation window.

7. The method of claim 5, wherein the first and second frames are adjacent frames in the sequence.

8. The method of claim 1, further comprising determining at least one of a lumen mask or a vessel mask using the at least one flow feature.

9. The method of claim 8, further comprising, for at least one of the one or more regions, determining at least one feature difference, in feature space, between the region and the lumen mask or the vessel mask utilizing the one or more texture features and the at least one flow feature determined for that region.

10. The method of claim 9, further comprising revising the lumen mask or the vessel mask using the at least one feature difference, wherein deriving the lumen border optionally comprises deriving the lumen border using the revised lumen mask or the revised vessel mask.

11. A non-transitory computer-readable medium having processor-executable instructions for processing a sequence of ultrasound frames, the processor-executable instructions when installed onto a device enable the device to perform actions, comprising:

   receiving a sequence of intravascular ultrasound, IVUS, frames of a vessel having a lumen, the sequence comprising a first frame and a second frame, wherein each frame comprises a plurality of scan lines, each scan line comprising a plurality of pixels;
   determining one or more texture features for each of one or more regions of the first frame, wherein at least one of the one or more texture features comprises a difference between intensity values of i) the region of the first frame and ii) another region of the first frame spaced apart from the region;
   determining at least one flow feature using cross-frame correlation for each of the one or more regions by comparing the first and second frames;
   deriving a lumen border for the first frame using the one or more texture features and the at least one flow feature to characterize the one or more regions as within or outside of the lumen of the vessel; and
   displaying an ultrasound image of the first frame with the lumen border,
   wherein determining at least one flow feature optionally comprises determining a cross-frame flow feature by determining a correlation value between a correlation window in the first frame and a search window in the second frame; and
   wherein the one or more regions are each a pixel of the first frame.

12. The non-transitory computer-readable medium of claim 11, wherein the actions further comprise
   determining at least one of a lumen mask or a vessel mask using the at least one flow feature;
   for at least one of the one or more regions, determining at least one feature difference, in feature space, between the region and the lumen mask or the vessel mask utilizing the one or more texture features and the at least one flow feature determined for that region; and
   revising the lumen mask or the vessel mask using the at least one feature difference;
   and wherein deriving the lumen border comprises deriving the lumen border using the revised lumen mask or the revised vessel mask.

13. A system (100) for generating and processing a sequence of ultrasound frames, comprising:

   a catheter (102);
   an ultrasound imaging core (306) insertable into the catheter (102), the ultrasound imaging core (306) comprising at least one transducer (312) and is configured and arranged for rotation of at least a portion of the ultrasound imaging core (306) to provide a sequence of ultrasound frames; and
   a processor (106), coupleable to the ultrasound imaging core (306), for executing processor-readable instructions that enable actions, including:

receiving a sequence of intravascular ultrasound, IVUS, frames of a vessel having a lumen, the sequence comprising a first frame and a second frame, wherein each frame comprises a plurality of scan lines, each scan line comprising a plurality of pixels;

determining one or more texture features for each of one or more regions of the first frame, wherein at least one of the one or more texture features comprises a difference between intensity values of i) the region of the first frame and ii) another region of the first frame spaced apart from the region;

determining at least one flow feature using cross-frame correlation for each of the one or more regions by comparing the first and second frames;

deriving a lumen border for the first frame using the one or more texture features and the at least one flow feature to characterize the one or more regions as within or outside of the lumen of the vessel; and

displaying an ultrasound image of the first frame with the lumen border,

wherein determining at least one flow feature optionally comprises determining a cross-frame flow feature by determining a correlation value between a correlation window in the first frame and a search window in the second frame; and

wherein the one or more regions are each a pixel of the first frame.

**14.** The system (100) of claim 13, wherein the actions further comprise

determining at least one of a lumen mask or a vessel mask using the at least one flow feature;

for at least one of the one or more regions, determining at least one feature difference, in feature space, between the region and the lumen mask or the vessel mask utilizing the one or more texture features and the at least one flow feature determined for that region; and

revising the lumen mask or the vessel mask using the at least one feature difference; and wherein deriving the lumen border comprises deriving the lumen border using the revised lumen mask or the revised vessel mask.

## Patentansprüche

**1.** Verfahren zur Verarbeitung einer Sequenz von Ultraschall-Einzelbildern zur Anzeige, wobei das Verfahren aufweist:

Empfangen einer Sequenz von Intravaskulär-Ultraschall (IVUS) -Einzelbildern eines Gefäßes mit einem Lumen, wobei die Sequenz ein erstes Einzelbild und ein zweites Einzelbild aufweist, wobei jedes Einzelbild mehrere Tastlinien aufweist, wobei jede Tastlinie mehrere Bildpunkte aufweist;

Bestimmen eines oder mehrerer Texturmerkmale für jeden von einem oder mehreren Bereichen des ersten Einzelbilds, wobei zumindest eines der einen oder mehreren Texturmerkmale eine Differenz zwischen Intensitätswerten von i) dem Bereich des ersten Einzelbilds und ii) einem von dem Bereich beabstandeten anderen Bereich des ersten Einzelbilds aufweist;

Bestimmen zumindest eines Flow-Merkmals mittels einer einzelbildübergreifenden Korrelation für jeden der einen oder mehreren Bereiche durch Vergleichen der ersten und zweiten Einzelbilder;

Herleiten einer Lumengrenze für das erste Einzelbild vermittels des einen oder der mehreren Texturmerkmale und des zumindest einen Flow-Merkmals, um den einen oder die mehreren Bereiche als innerhalb oder außerhalb des Lumens des Gefäßes zu kennzeichnen; und

Anzeigen eines Ultraschallbilds des ersten Einzelbilds mit der Lumengrenze;

wobei der eine oder die mehreren Bereiche jeweils ein Bildpunkt des ersten Einzelbilds sind.

**2.** Verfahren nach Anspruch 1, wobei das Bestimmen von einem oder mehr Texturmerkmalen das Bestimmen der Differenz von Intensitätswerten aus Bildpunkten von zwei oder mehr unterschiedlichen Tastlinien des ersten Einzelbilds aufweist, wobei das Bestimmen der Differenz der Intensitätswerte optional das Bestimmen der Differenz der Intensitätswerte aus Bildpunkten von zwei oder mehr unterschiedlichen, nicht-benachbarten Tastlinien aufweist.

**3.** Verfahren nach Anspruch 1, wobei das Bestimmen von einem oder mehreren Texturmerkmalen das Bestimmen der Differenz von Intensitätswerten aus zumindest zwei Bildpunkten einer einzelnen Tastlinie des ersten Einzelbilds aufweist, wobei das Bestimmen der Differenz von Intensitätswerten optional das Bestimmen der Differenz in Intensitätswerten von zumindest zwei nicht-benachbarten Bildpunkten einer einzelnen Tastlinie aufweist.

**4.** Verfahren nach Anspruch 1, wobei die Sequenz ferner zumindest ein zusätzliches Einzelbild aufweist, und das Verfahren ferner für jedes des zumindest einen zusätzlichen Einzelbilds aufweist:

Bestimmen von einem oder mehreren Texturmerkmalen für jeden von einem oder mehreren Bereichen des zusätzlichen Einzelbilds, wobei zumindest eines der einen oder mehreren Texturmerkmale eine Differenz zwischen Intensitätswerten von i) dem Bereich des zusätzlichen IVUS-Einzelbilds und ii) einem anderen, von dem Bereich beabstandeten Bereich des zusätzlichen IVUS-Einzelbilds aufweist;

Bestimmen zumindest eines Flow-Merkmals für jeden der einen oder mehreren Bereiche durch Vergleichen des zusätzlichen Einzelbilds mit einem anderen Einzelbild in der Sequenz; und

Herleiten einer Lumengrenze für das zusätzliche Einzelbild mittels der einen oder mehreren Texturmerkmale und des zumindest einen Flow-Merkmals, um die einen oder mehreren Bereiche als innerhalb oder außerhalb des Lumens des Gefäßes zu kennzeichnen; und

Anzeigen eines Ultraschallbilds des zusätzlichen Einzelbilds mit der Lumengrenze, die für das zusätzliche Einzelbild hergeleitet wurde;

wobei die einen oder mehreren Bereiche jeweils ein Bildpunkt sind.

5. Verfahren nach Anspruch 1, wobei das Bestimmen von zumindest einem Flow-Merkmal das Bestimmen eines einzelbildübergreifenden Flow-Merkmals durch Bestimmen eines Korrelationswerts zwischen einem Korrelationsfenster in dem ersten Einzelbild und einem Suchfenster in dem zweiten Einzelbild aufweist.

6. Verfahren nach Anspruch 5, wobei das Suchfenster eine größere Fläche als das Korrelationsfenster aufweist.

7. Verfahren nach Anspruch 5, wobei die ersten und zweiten Einzelbilder benachbarte Einzelbilder in der Sequenz sind.

8. Verfahren nach Anspruch 1, ferner aufweisend das Bestimmen einer Lumenmaske und/oder einer Gefäßmaske vermittels des zumindest einen Flow-Merkmals.

9. Verfahren nach Anspruch 8, ferner aufweisend, für zumindest einen der einen oder mehreren Bereiche, das Bestimmen von zumindest einer Merkmalsdifferenz, in einem Merkmalsraum, zwischen dem Bereich und der Lumenmaske oder der Gefäßmaske unter Verwendung der einen oder mehreren Texturmerkmale und des zumindest einen Flow-Merkmals, die für diesen Bereich bestimmt wurden.

10. Verfahren nach Anspruch 9, ferner aufweisend das Korrigieren der Lumenmaske oder der Gefäßmaske vermittels der zumindest einen Merkmalsdifferenz, wobei das Herleiten der Lumengrenze optional das Herleiten der Lumengrenze mittels der korrigierten Lumenmaske oder der korrigierten Gefäßmaske aufweist.

11. Nicht-flüchtiges, computerlesbares Medium mit durch einen Prozessor ausführbaren Anweisungen zur Verarbeitung einer Sequenz von Ultraschalleinzelbildern, wobei die durch einen Prozessor ausführbaren Anweisungen, wenn sie auf einer Vorrichtung installiert sind, die Vorrichtung in die Lage versetzen, Aktionen durchzuführen, aufweisend:

Empfangen einer Sequenz von Intravaskulär-Ultraschall (IVUS) -Einzelbildern eines Gefäßes mit einem Lumen, wobei die Sequenz ein erstes Einzelbild und ein zweites Einzelbild aufweist, wobei jedes Einzelbild mehrere Tastlinien aufweist, wobei jede Tastlinie mehrere Bildpunkte aufweist;

Bestimmen von einem oder mehreren Texturmerkmalen für jeden von einem oder mehreren Bereichen des ersten Einzelbilds, wobei zumindest eines der einen oder mehreren Texturmerkmale eine Differenz zwischen Intensitätswerten von i) dem Bereich des ersten Einzelbilds und ii) einem von dem Bereich beabstandeten anderen Bereich des ersten Einzelbilds aufweist;

Bestimmen zumindest eines Flow-Merkmals mittels einer einzelbildübergreifenden Korrelation für jeden der einen oder mehreren Bereiche durch Vergleichen der ersten und zweiten Einzelbilder;

Herleiten einer Lumengrenze für das erste Einzelbild vermittels des einen oder der mehreren Texturmerkmale und des zumindest einen Flow-Merkmals, um den einen oder die mehreren Bereiche als innerhalb oder außerhalb des Lumens des Gefäßes zu kennzeichnen; und

Anzeigen eines Ultraschallbilds des ersten Einzelbilds mit der Lumengrenze;

wobei das Bestimmen zumindest eines Flow-Merkmals optional das Bestimmen eines einzelbildübergreifenden Merkmals durch Bestimmen eines Korrelationswerts zwischen einem Korrelationsfenster in dem ersten Einzelbild und einem Suchfenster in dem zweiten Einzelbild aufweist; und

wobei der eine oder die mehreren Bereiche jeweils ein Bildpunkt des ersten Einzelbilds sind.

12. Nicht-flüchtiges computerlesbares Medium nach Anspruch 11, wobei die Aktionen ferner aufweisen:

Bestimmen einer Lumenmaske und/oder einer Gefäßmaske vermittels des zumindest einen Flow-Merkmals;
für zumindest einen der einen oder mehreren Bereiche, Bestimmen von zumindest einer Merkmalsdifferenz, in einem Merkmalsraum, zwischen dem Bereich und der Lumenmaske oder der Gefäßmaske unter Verwendung der einen oder mehreren Texturmerkmale und des zumindest einen Flow-Merkmals, die für diesen Bereich bestimmt wurden; und
Korrigieren der Lumenmaske oder der Gefäßmaske vermittels der zumindest einen Merkmalsdifferenz;
und wobei das Herleiten der Lumengrenze das Herleiten der Lumengrenze vermittels der korrigierten Lumenmaske oder der korrigierten Gefäßmaske aufweist.

**13.** System (100) zur Erzeugung und Verarbeitung einer Sequenz von Ultraschall-Einzelbildern, aufweisend:

einen Katheter (102);
einen Ultraschallbildgebungskem (306), der in den Katheter (102) einführbar ist,
wobei der Ultraschallbildgebungskem (306) zumindest einen Wandler (312) aufweist und zur Drehung von zumindest einem Abschnitt des Ultraschallbildgebungskems (306) eingerichtet und angeordnet ist, um eine Sequenz von Ultraschalleinzelbildern bereitzustellen; und
einen Prozessor (106), der an den Ultraschallbildgebungskem (306) koppelbar ist, zur Ausführung von Prozessor-lesbaren Anweisungen, die Aktionen ermöglichen, aufweisend:

Empfangen einer Sequenz von Intravaskulär-Ultraschall (IVUS) -Einzelbildern eines Gefäßes mit einem Lumen, wobei die Sequenz ein erstes Einzelbild und ein zweites Einzelbild aufweist, wobei jedes Einzelbild mehrere Tastlinien aufweist, wobei jede Tastlinie mehrere Bildpunkte aufweist;
Bestimmen von einem oder mehreren Texturmerkmalen für jeden von einem oder mehreren Bereichen des ersten Einzelbilds, wobei zumindest eines der einen oder mehreren Texturmerkmale eine Differenz zwischen Intensitätswerten von i) dem Bereich des ersten Einzelbilds und ii) einem von dem Bereich beabstandeten anderen Bereich des ersten Einzelbilds aufweist;
Bestimmen zumindest eines Flow-Merkmals mittels einer einzelbildübergreifen Korrelation für jeden der einen oder mehreren Bereiche durch Vergleichen der ersten und zweiten Einzelbilder;
Herleiten einer Lumengrenze für das erste Einzelbild vermittels des einen oder der mehreren Texturmerkmale und des zumindest einen Flow-Merkmals, um den einen oder die mehreren Bereiche als innerhalb oder außerhalb des Lumens des Gefäßes zu kennzeichnen; und
Anzeigen eines Ultraschallbilds des ersten Einzelbilds mit der Lumengrenze;
wobei das Bestimmen zumindest eines Flow-Merkmals optional das Bestimmen eines einzelbildübergreifenden Merkmals durch Bestimmen eines Korrelationswerts zwischen einem Korrelationsfenster in dem ersten Einzelbild und einem Suchfenster in dem zweiten Einzelbild aufweist; und
wobei der eine oder die mehreren Bereiche jeweils ein Bildpunkt des ersten Einzelbilds sind.

**14.** System (100) nach Anspruch 13, wobei die Aktionen ferner aufweisen:

Bestimmen einer Lumenmaske und/oder einer Gefäßmaske mittels des zumindest einen Flow-Merkmals;
für zumindest einen der einen oder mehreren Bereiche, Bestimmen von zumindest einer Merkmalsdifferenz, in einem Merkmalsraum, zwischen dem Bereich und der Lumenmaske oder der Gefäßmaske vermittels der einen oder mehreren Texturmerkmale und des zumindest einen Flow-Merkmals, die für diesen Bereich bestimmt wurden; und
Korrigieren der Lumenmaske oder der Gefäßmaske vermittels der zumindest einen Merkmalsdifferenz;
und wobei das Herleiten der Lumengrenze das Herleiten der Lumengrenze mittels der korrigierten Lumenmaske oder der korrigierten Gefäßmaske aufweist.

**Revendications**

**1.** Procédé de traitement d'une séquence de trames d'ultrasons à des fins d'affichage, le procédé comprenant les étapes ci-dessous consistant à :

recevoir une séquence de trames d'ultrasons intravasculaires, IVUS, d'un vaisseau présentant une lumière, la séquence comprenant une première trame et une seconde trame, dans lequel chaque trame comprend une pluralité de lignes de balayage, chaque ligne de balayage comprenant une pluralité de pixels ;
déterminer une ou plusieurs caractéristiques de texture pour chaque région parmi une ou plusieurs régions de la première trame, dans lequel au moins une caractéristique de texture de ladite une ou desdites plusieurs caractéristiques de texture comprend une différence entre des valeurs d'intensité i) de la région de la première trame et ii) d'une autre région de la première trame distante de la région ;

déterminer au moins une caractéristique d'écoulement en utilisant une corrélation de trames croisée pour chaque région de ladite une ou desdites plusieurs régions, en comparant les première et seconde trames ;

dériver une bordure de lumière pour la première trame en utilisant ladite une ou lesdites plusieurs caractéristiques de texture et ladite une ou lesdites plusieurs caractéristiques d'écoulement, en vue de caractériser ladite une ou lesdites plusieurs régions comme se situant à l'intérieur ou à l'extérieur de la lumière du vaisseau ; et

afficher une image ultrasonore de la première trame avec la bordure de lumière ;

dans lequel ladite une ou lesdites plusieurs régions correspondent chacune à un pixel de la première trame.

2. Procédé selon la revendication 1, dans lequel l'étape de détermination d'une ou plusieurs caractéristiques de texture consiste à déterminer la différence dans des valeurs d'intensité à partir de pixels de deux lignes de balayage différentes ou plus de la première trame, et dans lequel l'étape de détermination de la différence dans des valeurs d'intensité consiste facultativement à déterminer la différence de valeurs d'intensité à partir de pixels de deux lignes de balayage non adjacentes différentes ou plus.

3. Procédé selon la revendication 1, dans lequel l'étape de détermination d'une ou plusieurs caractéristiques de texture consiste à déterminer la différence dans des valeurs d'intensité à partir d'au moins deux pixels d'une unique ligne de balayage de la première trame, et dans lequel l'étape de détermination de la différence dans des valeurs d'intensité consiste facultativement à déterminer la différence dans des valeurs d'intensité à partir d'au moins deux pixels non adjacents d'une unique ligne de balayage.

4. Procédé selon la revendication 1, dans lequel la séquence comprend en outre au moins une trame supplémentaire, et le procédé comprend en outre, pour chaque trame de ladite au moins une trame supplémentaire, les étapes ci-dessous consistant à :

déterminer une ou plusieurs caractéristiques de texture pour chacune d'une ou plusieurs régions de la trame supplémentaire, dans lequel au moins une caractéristique de texture de ladite une ou desdites plusieurs caractéristiques de texture comprend une différence entre des valeurs d'intensité i) de la région de la trame IVUS supplémentaire et ii) d'une autre région de la trame IVUS supplémentaire distante de la région ;

déterminer au moins une caractéristique d'écoulement pour chaque région de ladite une ou desdites plusieurs régions, en comparant la trame supplémentaire à une autre trame dans la séquence ; et

dériver une bordure de lumière pour la trame supplémentaire, en utilisant ladite une ou lesdites plusieurs caractéristiques de texture et ladite au moins une caractéristique d'écoulement, en vue de caractériser ladite une ou lesdites plusieurs régions comme se situant à l'intérieur ou à l'extérieur de la lumière du vaisseau ; et afficher une image ultrasonore de la trame supplémentaire avec la bordure de lumière dérivée pour la trame supplémentaire ;

dans lequel ladite une ou lesdites plusieurs régions correspondent chacune à un pixel.

5. Procédé selon la revendication 1, dans lequel l'étape de détermination d'au moins une caractéristique d'écoulement consiste à déterminer une caractéristique d'écoulement de trames croisée en déterminant une valeur de corrélation entre une fenêtre de corrélation dans la première trame et une fenêtre de recherche dans la seconde trame.

6. Procédé selon la revendication 5, dans lequel la fenêtre de recherche présente une surface supérieure à celle de la fenêtre de corrélation.

7. Procédé selon la revendication 5, dans lequel les première et seconde trames sont des trames adjacentes dans la séquence.

8. Procédé selon la revendication 1, comprenant en outre l'étape consistant à déterminer au moins l'un parmi un masque de lumière ou un masque de vaisseau en utilisant ladite au moins une caractéristique d'écoulement.

9. Procédé selon la revendication 8, comprenant en outre, pour au moins une région parmi ladite une ou lesdites plusieurs régions, l'étape consistant à déterminer au moins une différence de caractéristique, dans un espace de caractéristique, entre la région et le masque de lumière ou le masque de vaisseau, en utilisant ladite une ou lesdites plusieurs caractéristiques de texture et ladite au moins une caractéristique d'écoulement déterminée pour cette région.

**10.** Procédé selon la revendication 9, comprenant en outre l'étape consistant à corriger le masque de lumière ou le masque de vaisseau en utilisant ladite au moins une différence de caractéristique, dans lequel l'étape de dérivation de la bordure de lumière consiste facultativement à dériver la bordure de lumière en utilisant le masque de lumière corrigé ou le masque de vaisseau corrigé.

**11.** Support non transitoire lisible par ordinateur présentant des instructions exécutables par processeur pour traiter une séquence de trames d'ultrasons, les instructions exécutables par processeur, lorsqu'elles sont installées sur un dispositif, permettant au dispositif de mettre en oeuvre des actions, comprenant les étapes ci-dessous consistant à :

recevoir une séquence de trames d'ultrasons intravasculaires, IVUS, d'un vaisseau présentant une lumière, la séquence comprenant une première trame et une seconde trame, dans lequel chaque trame comprend une pluralité de lignes de balayage, chaque ligne de balayage comprenant une pluralité de pixels ;
déterminer une ou plusieurs caractéristiques de texture pour chaque région parmi une ou plusieurs régions de la première trame, dans lequel au moins une caractéristique de texture de ladite une ou desdites plusieurs caractéristiques de texture comprend une différence entre des valeurs d'intensité i) de la région de la première trame et ii) d'une autre région de la première trame distante de la région ;
déterminer au moins une caractéristique d'écoulement en utilisant une corrélation de trames croisée pour chaque région de ladite une ou desdites plusieurs régions, en comparant les première et seconde trames ;
dériver une bordure de lumière pour la première trame en utilisant ladite une ou lesdites plusieurs caractéristiques de texture et ladite une ou lesdites plusieurs caractéristiques d'écoulement, en vue de caractériser ladite une ou lesdites plusieurs régions comme se situant à l'intérieur ou à l'extérieur de la lumière du vaisseau ; et
afficher une image ultrasonore de la première trame avec la bordure de lumière ;
dans lequel l'étape de détermination d'au moins une caractéristique d'écoulement consiste facultativement à déterminer une caractéristique d'écoulement de trames croisée, en déterminant une valeur de corrélation entre une fenêtre de corrélation dans la première trame et une fenêtre de recherche dans la seconde trame ; et
dans lequel ladite une ou lesdites plusieurs régions correspondent chacune à un pixel de la première trame.

**12.** Support non transitoire lisible par ordinateur selon la revendication 11, dans lequel les actions comprennent en outre les étapes ci-dessous consistant à :

déterminer au moins l'un parmi un masque de lumière ou un masque de vaisseau en utilisant ladite au moins une caractéristique d'écoulement ;
pour au moins une région parmi ladite une ou lesdites plusieurs régions, déterminer au moins une différence de caractéristique, dans un espace de caractéristique, entre la région et le masque de lumière ou le masque de vaisseau, en utilisant ladite une ou lesdites plusieurs caractéristiques de texture et ladite au moins une caractéristique d'écoulement déterminée pour cette région ; et
corriger le masque de lumière ou le masque de vaisseau en utilisant ladite au moins une différence de caractéristique ; et
dans lequel l'étape de dérivation de la bordure de lumière consiste à dériver la bordure de lumière en utilisant le masque de lumière corrigé ou le masque de vaisseau corrigé.

**13.** Système (100) destiné à générer et à traiter une séquence de trames d'ultrasons, comprenant :

un cathéter (102) ;
un noyau d'imagerie par ultrasons (306) pouvant être inséré dans le cathéter (102), le noyau d'imagerie par ultrasons (306) comprenant au moins un transducteur (312), et étant configuré et agencé de manière à faire tourner au moins une partie du noyau d'imagerie par ultrasons (306) en vue de fournir une séquence de trames d'ultrasons ; et
un processeur (106), pouvant être couplé au noyau d'imagerie par ultrasons (306), en vue d'exécuter des instructions lisibles par processeur qui permettent des actions comprenant les étapes ci-dessous consistant à :

recevoir une séquence de trames d'ultrasons intravasculaires, IVUS, d'un vaisseau présentant une lumière, la séquence comprenant une première trame et une seconde trame, dans lequel chaque trame comprend une pluralité de lignes de balayage, chaque ligne de balayage comprenant une pluralité de pixels ;
déterminer une ou plusieurs caractéristiques de texture pour chaque région parmi une ou plusieurs régions de la première trame, dans lequel au moins une caractéristique de texture de ladite une ou desdites plusieurs caractéristiques de texture comprend une différence entre des valeurs d'intensité i) de la région de la première trame et ii) d'une autre région de la première trame distante de la région ;

déterminer au moins une caractéristique d'écoulement en utilisant une corrélation de trames croisée pour chaque région de ladite une ou desdites plusieurs régions, en comparant les première et seconde trames ;

dériver une bordure de lumière pour la première trame en utilisant ladite une ou lesdites plusieurs caractéristiques de texture et ladite une ou lesdites plusieurs caractéristiques d'écoulement, en vue de caractériser ladite une ou lesdites plusieurs régions comme se situant à l'intérieur ou à l'extérieur de la lumière du vaisseau ; et

afficher une image ultrasonore de la première trame avec la bordure de lumière ;

dans lequel l'étape de détermination d'au moins une caractéristique d'écoulement consiste facultativement à déterminer une caractéristique d'écoulement de trames croisée en déterminant une valeur de corrélation entre une fenêtre de corrélation dans la première trame et une fenêtre de recherche dans la seconde trame ; et

dans lequel ladite une ou lesdites plusieurs régions correspondent chacune à un pixel de la première trame.

**14.** Système (100) selon la revendication 13, dans lequel les actions comprennent en outre les étapes ci-dessous consistant à :

déterminer au moins l'un parmi un masque de lumière ou un masque de vaisseau en utilisant ladite au moins une caractéristique d'écoulement ;

pour au moins une région parmi ladite une ou lesdites plusieurs régions, déterminer au moins une différence de caractéristique, dans un espace de caractéristique, entre la région et le masque de lumière ou le masque de vaisseau, en utilisant ladite une ou lesdites plusieurs caractéristiques de texture et ladite au moins une caractéristique d'écoulement déterminée pour cette région ; et

corriger le masque de lumière ou le masque de vaisseau en utilisant ladite au moins une différence de caractéristique ; et

dans lequel l'étape de dérivation de la bordure de lumière consiste à dériver la bordure de lumière en utilisant le masque de lumière corrigé ou le masque de vaisseau corrigé.

**Fig. 1**

**Fig. 2**

**Fig. 3**

**Fig. 4**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6152878 A
- US 6945938 B
- US 7246959 B
- US 7306561 B
- US 20060100522 A
- US 20060106320 A
- US 20060173350 A
- US 20060253028 A
- US 20070016054 A
- US 20070038111 A